Europäisches Patentamt

European Patent Office

Office européen des brevets

(19)

(11) Publication number : **0 474 364 A2**

# EUROPEAN PATENT APPLICATION

(12)

(21) Application number : **91307207.0**

(22) Date of filing : **06.08.91**

(51) Int. Cl.⁵ : **G06K 9/82,** G06K 9/68

(30) Priority : **10.08.90 GB 9017570**

(43) Date of publication of application :
**11.03.92 Bulletin 92/11**

(84) Designated Contracting States :
**CH DE FR LI**

(71) Applicant : **GEC-MARCONI LIMITED**
**The Grove, Warren Lane**
**Stanmore, Middlesex HA7 4LY (GB)**

(72) Inventor : **Webster, Simon Charles**
**5 Torridge Walk**
**Hemel Hempstead, Hertfordshire HP2 6ET**
**(GB)**

(74) Representative : **George, Sidney Arthur**
**The General Electric Company p.l.c. GEC**
**Patent Department(Wembley Office) Hirst**
**Research Centre East Lane**
**Wembley Middlesex, HA9 7PP (GB)**

(54) **Pattern matching apparatus.**

(57) Apparatus for comparing an input genome sequence with one or more reference sequences comprises signal generators (10) for generating an input signal for each occurrence of each base component type (A,C,G,T) of the input sequence. A respective optical correlator (23-26) is provided for each base component type. Each correlator detects a match or near match between the occurrences of the respective base component type in the input sequence and the occurrences of that component type in any of the reference sequences, and produces a respective correlation signal. Decision-making circuitry (31) responds to the correlation signals from all of the correlators to provide an indication of a match or near match between the whole input sequence and any of the reference sequences.

EP 0 474 364 A2

# Fig.2.

This invention relates to pattern matching apparatus, and particularly to apparatus for matching genome sequences.

A genome sequence is a one-dimensional code which represents the order of base components in deoxyribonucleic acid (DNA) and ribonucleic acid (RNA). Such sequences can be used to study hereditary characteristics in animals and plants. The base components are represented by their initial letters A,C,G and T, respectively, for DNA, the letter T being replaced by the letter U for RNA. The genome sequences may be very long, e.g. of the order of billions of bases. An example of a small section of a DNA sequence might be A-A-C-C-A-T-A-G....

There is a need to carry out pattern recognition techniques to detect the presence and the location of sections of a particular sequence in a full DNA or RNA code.

It is an object of the present invention to provide apparatus for comparing actually-occurring genome sequences with one or more reference sequences to detect a match or a near-match therebetween.

According to the invention there is provided apparatus for comparing an input genome sequence, comprising a plurality of base component types, with one or more reference sequences to detect a match or near match therebetween, the apparatus comprising means to generate a respective input signal for each base component type, the input signal indicating each occcurrence of the respective base component type in the input sequence; respective optical correlator means for each base component type, each correlator means being responsive to the respective input signal to detect a match or near match between the occurrences of the respective base component type in the input sequence and occurrences of that component type in the reference sequence or any of said reference sequences and to produce in response thereto a respective correlation signal; and means responsive to the correlation signals from all of said correlator means to provide an indication of a match or near match between said input sequence and said reference sequence or any of said reference sequences.

Embodiments of the invention will now be described, by way of example, with reference to the accompanying drawings, in which

Figure 1 is a schematic pictorial view of apparatus for effecting a known optical one-dimensional correlation technique,

Figure 2 is a block schematic diagram of one form of genome pattern matching apparatus in accordance with the invention, and

Figure 3 is a block schematic diagram of a second form of genome pattern matching apparatus in accordance with the invention.

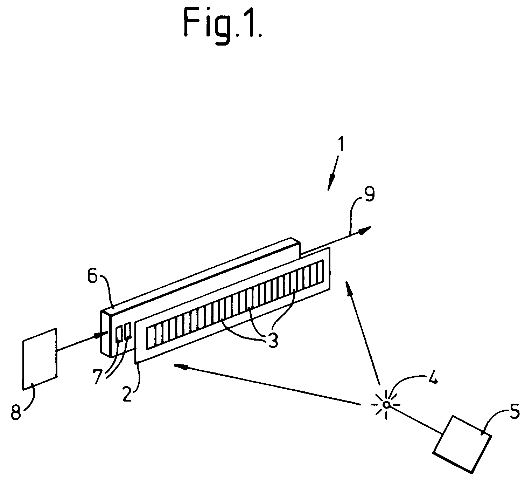

Referring to Figure 1 of the drawings, apparatus 1 for effecting a known one-dimensional correlation technique comprises a mask 2 which is formed with successive elements 3 along its length having different light transmission values representing a reference data sequence. The mask 2 is iluminated by a light source 4, which may comprise, for example, one or more light-emitting diodes. The intensity of the light from the source 4 is modulated, from instant to instant, by a supply 5, in accordance with an input data sequence. A linear array 6 of CCD devices 7 is located behind the mask 2, with a respective device 7 aligned with each element 3. The charge on the devices is shifted along the array, from left to right in the figure, by clock pulses from a clock generator 8, in synchronism with the input rate of the data modulating the light source 4.

The apparatus operates on a time delay and integration (TDI) process, providing cross correlation between the input data and the reference data by producing output current maxima on an output line 9 whenever matching of input and reference data bits is found. The temporal positions of the maxima in the correlation output signal on the line 9 indicate the times at which correspondence between the input data and the reference data has occurred. In this way, correspondence between a number of successive data input bits and successive reference data bits can be detected. The mask 2 can be readily replaced by another bearing different reference data. Alternatively, the mask may be constituted by electrically-updateable elements or an electrically-addressed spatial light modulator.

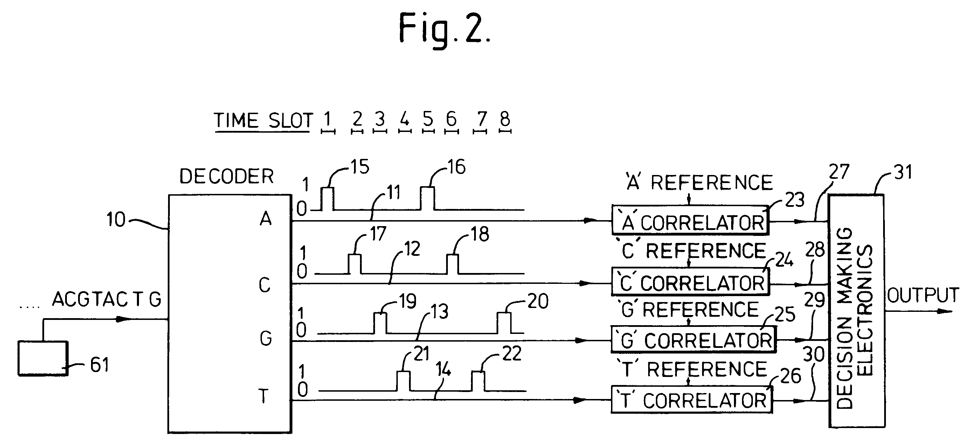

An extension of this correlation technique is used in a first embodiment of the invention. Referring to Figure 2 of the drawings, an input signal representing a test DNA sequence is fed into a decoder 10 from source 61. A section of the sequence of base components A,C,G and T may be, for example, ----ACGTACTG as shown, the left-hand component in the sequence being entered first into the decoder 10. The decoder separates the A-representing signals from the C-representing signals, and so on, and provides respective A,C,G and T binary "1" pulses on output lines 11,12,13 and 14 when those base components appear in the decoder input. For the sequence section shown, there will be two pulses 15 and 16 in first and fifth time slots on the "A" line 11; pulses 17 and 18 in second and sixth time slots on the "C" line 12; pulses 19 and 20 in third and eighth time slots on the "G" line 13; and pulses 21 and 22 in fourth and seventh time slots on the "T" line 14.

The pulses on the lines 11-14 are fed to respective correlators 23-26, each of which may be similar to the TDI apparatus 1 of Figure 1. In each such correlator the pulses on the respective line 11-14 will be used to modulate a light source in the correlator, and an A,C,G or T reference sequence, as the case may be, will be stored in the correlator mask. The accumulated charge will be stepped along the respective CCD array in synchronism with the overall data rate on the lines 11-14, i.e. in synchronism with the passing of the time slots 1-8.

Each correlator 23-26 will produce an output signal, on a respective output line 27-30, containing maxima corresponding to correlation between the A,C,G or T components in the input sequence and in the respective reference sequence.

The signals on the lines 27-30 are fed to a decision-making circuit 31 which produces an output having maxima the magnitude of which indicates the closeness of matching between the sequences, and the timing of which indicates the locations of the matching sections of the sequences. The circuit 31 compares the signal on each of its input lines 27-30 with a threshold value, performs the weighted sum of the thresholded signals, and compares the result with a threshold value. The threshold values and/or the weights can be set to give a greater significance to a match in some channels (i.e. the A,C,G and T correlator channels ) than in others.

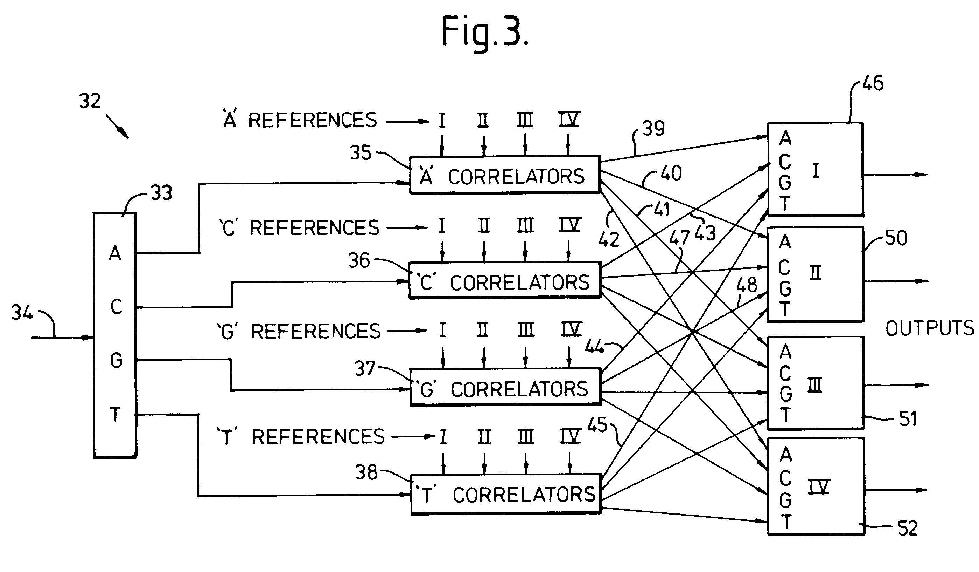

Figure 3 illustrates an alternative embodiment of the invention, in which a genome pattern matching apparatus 32 comprises a decoder 33, similar to the decoder 10 of Figure 2, to which an input DNA sequence is fed via a line 34. The A,C,G and T outputs of the decoder 33 are fed to respective correlators 35-38, as before,but in this case each correlator has a plurality of reference sequences. For example, each correlator is shown as having four reference sequences as I, II, III and IV, such that the sequences I of the four correlators together consitute a first reference DNA sequence section; the reference sequences II together constitute a second, different, reference DNA sequence section, and so on.

The "A" channel correlator 35 comprises four masks or spatial light modulators, each associated with a respective row of CCDs of a two-dimensional CCD array. As each "A" input pulse is received by the correlator 35, the input pulse causes modulation of the output of four light sources, each associated with a respective one of the masks. The correlator therefore produces four "A" correlation signals which are fed out via respective output lines 39-42.

The C, G and T correlators 36,37 and 38, respectively, operate in the same manner, so that each produces four outputs.

The output on the line 39 and the outputs from the correlators 36, 37, and 38 on lines 43,44 and 45, respectively, all relating to the reference sequence I, are fed to a "sequence I" decision-making circuit 46. Similarly, the outputs on lines 40,47,48 and 49 relating to the reference sequence II are fed to a decision-making circuit 50. Likewise, the outputs relating to the reference sequences III and IV are fed to decision-making circuits 51 and 52. The circuits 46,50,51 and 52 all act in a similar manner to the circuit 31 of Figure 2 to produce outputs relating to matching or near matching of the input DNA sequence section with the respective reference sequence section I, II, III or IV. A number of reference sequence sections can thereby be searched for during a single pass of the input DNA sequence.

It will be apparent that any other number of reference sequence sections could be searched for simultaneously by increasing or decreasing the number of reference masks and asscociated components in the correlators and correspondingly increasing or decreasing the number of decison-making circuits.

The apparatus of the present invention would be used to find matches (or near matches) of a test sequence with a reference sequence. The optical processor is much faster than an electronic counterpart, so rapid matching is possible. Once a match or near-match is found, a more versatile all-electronic procedure might be used to analyse the matching part of the test sequence in more detail.

The input to the decoder 10 or 34 could be taken from a computer memory such as a disc drive, or from a data communications link.

By converting the input data to a multichannel binary sequence, rather than correlating analogue information (such as representing A, C, G and T as values of 0.25, 0.5, 0.75 and 1.0, for example), the level of the cross-correlation signal for non-matching codes is reduced with respect to the near-matching or matching cases, thereby increasing the discrimination of the system.

Although the apparatus has been described in relation to DNA sequences, it can clearly be used for matching RNA sequences or other sequences of a similar format.

## Claims

1. Apparatus for comparing an input genome sequence, comprising a plurality of base component types (A,C,G,T), with one or more reference sequences to detect a match or near match therebetween, characterised by means (10;33) to generate a respective input signal (11-14) for each base component type, the input signal indicating each occcurrence of the respective base component type in the input sequence; respective optical correlator means (23-26; 35-38) for each base component type, each correlator means being responsive to the respective input signal to detect a match or near match between the occurrences of the respective base component type in the input sequence and occurrences of that component type in the reference sequence or any of said reference sequences and to produce in response thereto a respective correlation signal; and means (31;46,50,51,52) responsive to the correlation signals from all of said correlator means to provide an indication of a match or near match between said input sequence and said reference sequence or any of said reference sequences.

2. Apparatus as claimed in Claim 1, characterised in that each correlator means (23-26; 35-38) comprises optical storage means (2) having storage elements (3) for storing an optical representation of respective occurrences of the respective base component type (A,C,G,T) in the reference sequence; a light source (4) for illuminating the optical storage means, the light output of the light source being modulated in response to the respective input signal; and an array (6) of charge-coupled devices (7) for receiving the modulated light after passage through the storage means.

3. Apparatus as claimed in Claim 2, characterised in that the optical storage means (2) comprises a mask having areas the light transmittance of which provides said optical representation.

4. Apparatus as claimed in Claim 2, characterised in that the optical storage means (2) comprises a spatial light modulator.

5. Apparatus as claimed in any preceding claim, characterised in that each said input signal comprises a sequence of binary pulses.

6. Apparatus as claimed in any preceding claim, characterised in that the means (31;46,50,51,52) responsive to the correlation signals is operative to give more significance to a correlation signal from one correlator means (23-26;35-38) than from another.

7. Apparatus as claimed in any preceding claim, characterised by a plurality of said correlator means (35-38) for each base component type (A,C,G,T), each correlator means for a base component type being related to a different reference sequence.

# Fig.1.

# Fig. 2.

TIME SLOT 1 2 3 4 5 6 7 8

DECODER

.... ACGTACT G

10

61

A 15 11 16
C 17 12 18
G 19 13 20
T 21 14 22

'A' REFERENCE
'A' CORRELATOR 23

'C' REFERENCE
'C' CORRELATOR 24

'G' REFERENCE
'G' CORRELATOR 25

'T' REFERENCE
'T' CORRELATOR 26

27 31
28
29
30

DECISION MAKING ELECTRONICS

OUTPUT

EP 0 474 364 A2

# Fig.3.

'A' REFERENCES → I  II  III  IV

'C' REFERENCES → I  II  III  IV

'G' REFERENCES → I  II  III  IV

'T' REFERENCES → I  II  III  IV

OUTPUTS

EP 0 474 364 A2